# EUROPEAN PATENT APPLICATION

(11) **EP 4 682 161 A1**
(43) Date of publication of application: **21.01.2026**
(21) Application number: 24774025.1
(22) Date of filing: 15.03.2024
(51) Int. Cl.: C07K 14/34, C12N 15/31, C12N 15/77, C12N 1/21, C12P 13/08

(54) **TKT GENE MUTANT AND APPLICATION THEREOF IN PREPARATION OF L-LYSINE**

(30) Priority: 17.03.2023 CN 202310261458
(71) Applicant: Heilongjiang Eppen Biotech Co., Ltd., Daqing, Heilongjiang 166200 (CN)
(72) Inventor: WEI, Aiying, Daqing, Heilongjiang 166200 (CN); MENG, Gang, Daqing, Heilongjiang 166200 (CN); MA, Wenyou, Daqing, Heilongjiang 166200 (CN); ZHOU, Xiaoqun, Daqing, Heilongjiang 166200 (CN); MA, Fengyong, Daqing, Heilongjiang 166200 (CN); ZHAO, Chunguang, Daqing, Heilongjiang 166200 (CN); TIAN, Bin, Daqing, Heilongjiang 166200 (CN); WANG, Jiwei, Daqing, Heilongjiang 166200 (CN); MA, Congling, Daqing, Heilongjiang 166200 (CN)
(74) Representative: Winter, Brandl - Partnerschaft mbB
(86) International application number: PCT/CN2024/081895
(87) International publication number: WO 2024/193460

(57) **Abstract**

Provided are a TKT gene mutant and an application thereof in the preparation of L-lysine. The TKT gene mutant is a DNA molecule as represented by SEQ ID NO: 3, 5, 7, 9, 11, or 13 in the sequence listing, which encodes a protein as represented by SEQ ID NO: 4, 6, 8, 10, 12, or 14, and a wild type TKT gene is a DNA molecule as represented by SEQ ID NO: 1, which encodes a protein represented by SEQ ID NO: 2. It has been experimentally verified that the TKT gene and the mutant thereof can improve the yield of L-lysine and can be used for the production of L-lysine, and same have good application prospects.

## Description

### Cross references of related applications

The present application claims the priority to Chinese patent application (application number: 202310261458.1), filed on March 17, 2023, the entire content of which is incorporated herein by reference.

### Technical Field

The present invention relates to the field of biotechnology, and more particularly, to a TKT gene mutant and an application thereof in the preparation of L-lysine.

### Background Art

L-lysine has physiological effects such as promoting development, enhancing immunity and improving central nervous system functions. It is one of the eight essential amino acids that humans and animals cannot synthesize on their own and are necessary for growth. At present, L-lysine, as the second largest amino acid variety in the world, is mainly produced by a fermentation method, wherein *Corynebacterium glutamicum* is an important production strain for lysine. L-lysine accounting for approximately 90% of the industrial output is used as a nutritional enhancer in the feed industry, 10% as an umami agent and a sweetener in the food industry, as well as a drug intermediate in the pharmaceutical industry.

Improvements to the production of L-lysine by the fermentation method may involve fermentation techniques such as agitation and oxygen supply; or involve the composition of a nutrient medium, such as sugar concentration during fermentation; or involves processing fermentation broth into suitable product forms, e.g., by drying and granulating fermentation broth or ion exchange chromatography; or involve the intrinsic performance properties of related microorganisms themselves.

Methods used to improve the performance properties of these microorganisms include mutagenesis, mutant selection, and screening. Strains obtained in this way are resistant to antimetabolites or are auxotrophic for metabolites that are of regulatory importance and produce L-lysine.

### Summary of the Invention

An object of the present invention is to provide a protein that can be used for the production of L-lysine, the protein being named as a TKT protein, and the TKT protein being A1) or A2) or A3) as follows:
A1) a protein comprising (or being) SEQ ID No. 2, or a mutant protein which is obtained by mutating an alanine residue at position 327 in SEQ ID No. 2 into a threonine residue, a serine residue, a cysteine residue, a proline residue, an asparagine residue, a glutamine residue, a phenylalanine residue, a leucine residue, a valine residue, an isoleucine residue, an aspartic acid residue, a methionine residue, an arginine residue, a glutamic acid residue, a glycine residue, a histidine residue, a lysine residue, a tryptophan residue, or a tyrosine residue;
A2) a protein which is obtained by substitution and/or deletion and/or addition of one or more amino acid residues in an amino acid sequence of the protein of A1) except for position 327 and has the same function as A1); and
A3) a fusion protein obtained by linking a tag to an N-terminal or/and a C-terminal of A1) or A2). To facilitate purification of the protein of A1), the following tag Poly-Arg, Poly-His, FLAG, Strep-tag II or c-myc may be linked to an amino terminal or a carboxyl terminal of the protein.

The above protein of A2) is a protein that has 75% or more identity with an amino acid sequence of the protein of A1) and has the same function as A1). The identity of 75% or more may be the identity of 75%, 80%, 85%, 90%, 95%, 96%, 97%, 98% or 99%.

The above protein of A2) may be synthesized artificially, or may be obtained by synthesizing its encoding gene first and then performing biological expression.

The encoding gene of the above protein of A2) may be obtained by deletion of codons of one or more amino acid residues in a DNA sequence as represented by SEQ ID No. 3, SEQ ID No. 5, SEQ ID No. 7, SEQ ID No. 9, SEQ ID No. 11, SEQ ID No. 13 or SEQ ID No. 1, and/or missense mutations of one or more base pairs, and/or by linking encoding sequences of tags shown in the above table to a 5' end and/or a 3' end of the protein. Wherein, the DNA molecules as represented by SEQ ID No. 3, SEQ ID No. 5, SEQ ID No. 7, SEQ ID No. 9, SEQ ID No. 11, SEQ ID No. 13 and SEQ ID No. 1 encode proteins as represented by SEQ ID No. 4, SEQ ID No. 6, SEQ ID No. 8, SEQ ID No. 10, SEQ ID No. 12, SEQ ID No. 14, and SEQ ID No. 2, respectively.

The present invention further provides a biomaterial associated with a TKT protein. The biomaterial is any of B1) to B4) as follows:
B1) a nucleic acid molecule that encodes the TKT protein;
B2) an expression cassette containing the nucleic acid molecule of B1);
B3) a recombinant vector containing the nucleic acid molecule of B1), or a recombinant vector containing the expression cassette of B2); and
B4) a recombinant microorganism containing the nucleic acid molecule of B1), or a recombinant microorganism containing the expression cassette of B2, or a recombinant microorganism containing the recombinant vector of B3).

In the above biomaterial, the nucleic acid molecule of B1) may be any of b11)-b19) as follows:
b11) a DNA molecule as represented by SEQ ID No. 3 in a sequence listing;
b12) a DNA molecule as represented by SEQ ID No. 5 in the sequence listing;
b13) a DNA molecule as represented by SEQ ID No. 7 in the sequence listing;
b14) a DNA molecule as represented by SEQ ID No. 9 in the sequence listing;
b15) a DNA molecule as represented by SEQ ID No. 11 in the sequence listing;
b16) a DNA molecule as represented by SEQ ID No. 13 in the sequence listing;
b17) a DNA molecule as represented by SEQ ID No. 1 in the sequence listing;
b18) a DNA molecule that has the identity of 75% or more with a nucleotide sequence defined by any of b11)- b17) and encodes the TKT protein; and
b19) a genomic DNA molecule that hybridizes with the nucleotide sequence defined by any of b11)- b18) under stringent conditions and encodes the TKT protein.

The nucleic acid molecule may be DNA, such as cDNA, genomic DNA, or recombinant DNA; and the nucleic acid molecule may also be RNA, such as mRNA or hnRNA.

A person of ordinary skill in the art can readily mutate the nucleotide sequence that encodes the TKT protein of the present invention by using known methods, such as directed evolution or point mutation. Those artificially modified nucleotides that have the identity of 75% or more with the nucleotide sequence of the TKT protein of the present invention, as long as they are capable of encoding the TKT protein and have a function of the TKT protein, are all derived from the nucleotide sequences of the present invention and are equivalent to the sequences of the present invention.

The term "identity" as used herein refers to a sequence similarity to a natural nucleic acid sequence. "Identity" includes a nucleotide sequence that has the identity of 75% or more, or 85% or more, or 90% or more, or 95% or more with the nucleotide sequence that encodes the TKT protein of the present invention. The identity may be evaluated with the naked eyes or by computer software. Using the computer software, the identity between two or more sequences may be expressed by percentage (%), which may be used to evaluate the identity between related sequences.

In the above biomaterial, the stringent conditions may be as follows: hybridizing at 50°C in a mixed solution of 7% sodium dodecyl sulfate (SDS), 0.5 M NaPO₄ and 1 mM EDTA, and rinsing at 50°C in 2×SSC and 0.1% SDS; or hybridizing at 50°C in a mixed solution of 7% SDS, 0.5 M NaPO₄ and 1 mM EDTA, and rinsing at 50°C in 1×SSC and 0.1% SDS; or hybridizing at 50°C in a mixed solution of 7% SDS, 0.5 M NaPO₄ and 1 mM EDTA, and rinsing at 50°C in 0.5×SSC and 0.1% SDS; or hybridizing at 50°C in a mixed solution of 7% SDS, 0.5 M NaPO₄ and 1 mM EDTA, and rinsing at 50°C in 0.1×SSC and 0.1% SDS; or hybridizing at 50°C in a mixed solution of 7% SDS, 0.5 M NaPO₄ and 1 mM EDTA, and rinsing at 65°C in 0.1×SSC and 0.1% SDS; or hybridizing at 65°C in a solution of 6×SSC and 0.5% SDS, and then washing a film once with 2×SSC and 0.1% SDS, and 1×SSC and 0.1% SDS, respectively; or hybridizing at 68°C in a solution of 2×SSC and 0.1% SDS, and washing a film twice, 5 min each time, and then hybridizing at 68°C in a solution of 0.5×SSC and 0.1% SDS, and washing a film twice, 15 min each time; or hybridizing at 65°C in a solution of 0.1×SSPE (or 0.1×SSC) and 0.1% SDS, and washing a film.

The above identity of 75% or more may be the identity of 80%, 85%, 90% or 95% or more.

In the above biomaterial, the expression cassette (TKT gene expression cassette) of B2), which contains the nucleic acid molecule that encodes the TKT protein, refers to DNA capable of expressing the TKT protein in a host cell; and this DNA may not only include a promoter that initiates the transcription of a TKT gene, but also a terminator that terminates the transcription of the TKT gene. Further, the expression cassette may also include an enhancer sequence.

In the above biomaterial, the promoter in the expression cassette of B2) is a DNA molecule shown by positions 35-437 in SEQ ID No. 15.

In the above biomaterial, the vector may be a plasmid, a cosmid, a bacteriophage or a viral vector. The plasmid may be specifically a pXMJ19 or pK18mobsacB plasmid.

The recombinant vector of B3) may be pXMJ19-TKT, pXMJ19-TKT^{A327T}, pXMJ19-TKT^{A327S}, pXMJ19-TKT^{A327C}, pXMJ19-TKT^{A327P}, pXMJ19-TKT^{A327N}, pXMJ19-TKT^{A327Q} or pK18-TKT^{A327T}.
pXMJ19-TKT is a recombinant vector obtained by replacing a DNA fragment between Xbal I and BamH I recognition sequences of pXMJ19 with a DNA fragment as represented by SEQ ID No. 15;
pXMJ19-TKT^{A327T} differs from pXMJ19-TKT in that pXMJ19-TKT^{A327T}is a recombinant vector which is obtained by replacing a gene as represented by SEQ ID No. 1 in pXMJ19-TKT with a gene as represented by SEQ ID No. 3;
pXMJ19-TKT^{A327S} differs from pXMJ19-TKT in that pXMJ19-TKT^{A327S}is a recombinant vector which is obtained by replacing the gene as represented by SEQ ID No. 1 in pXMJ19-TKT with a gene as represented by SEQ ID No. 5;
pXMJ19-TKT^{A327C} differs from pXMJ19-TKT in that pXMJ19-TKT^{A327C}is a recombinant vector which is obtained by replacing the gene as represented by SEQ ID No. 1 in pXMJ19-TKT with a gene as represented by SEQ ID No. 7;
pXMJ19-TKT^{A327P} differs from pXMJ19-TKT in that pXMJ19-TKT^{A327P}is a recombinant vector which is obtained by replacing the gene as represented by SEQ ID No. 1 in pXMJ19-TKT with a gene as represented by SEQ ID No. 9;
pXMJ19-TKT^{A327N} differs from pXMJ19-TKT in that pXMJ19-TKT^{A327N} is a recombinant vector which is obtained by replacing the gene as represented by SEQ ID No. 1 in pXMJ19-TKT with a gene as represented by SEQ ID No. 11;
pXMJ19-TKT^{A327Q} differs from pXMJ19-TKT in that pXMJ19-TKT^{A327Q} is a recombinant vector which is obtained by replacing the gene as represented by SEQ ID No. 1 in pXMJ19-TKT with a gene as represented by SEQ ID No. 13; and
pK18-TKT^{A327T} is a recombinant vector which is obtained by replacing a fragment (small fragment) between Xbal I and BamH I recognition sites of a pK18mobsacB vector with a DNA fragment as represented by SEQ ID No. 6 in the sequence listing, and keeping other sequences of the pK18mobsacB vector unchanged.

In the above biomaterial, the microorganism may be yeast, bacteria, algae, or fungus. The bacteria may be *Escherichia coli, Corynebacterium glutamicum, brevibacterium lactofermentum, Corynebacterium pekinense, Brevibacterium ammoniagenes, Corynebacterium crenatum, Pantoea, Pantoea ananatis, Bacillus brevis, Lactobacillus brevis* or *Brevibacterium flavum.* The yeast may be *Saccharomyces cerevisiae* or *Pichia pastoris.*

In an example of the present invention, the *Corynebacterium glutamicum* is *Corynebacterium glutamicum* YP097158 or *Corynebacterium glutamicum* ATCC13032.

The recombinant microorganism of B4) is a recombinant microorganism which is obtained by replacing a TKT gene in the microorganism containing the tkt gene as represented by SEQ ID No. 1 with that as represented by SEQ ID No. 3, SEQ ID No. 5, SEQ ID No. 7, SEQ ID No. 9, SEQ ID No. 11 or SEQ ID No. 13, or a recombinant microorganism which is obtained by introducing the nucleic acid molecule of B1) into the microorganism and expressing the nucleic acid molecule of B1).

In an example of the present invention, the recombinant microorganism is recombinant bacteria ATCC13032-pXMJ19-TKT^{A327T}, ATCC13032-pXMJ19-TKT^{A327S}, ATCC13032-pXMJ19-TKT^{A327C}, ATCC13032-pXMJ19-TKT^{A327P}, ATCC13032-pXMJ19-TKT^{A327N}, ATCC13032-pXMJ19-TKT^{A327Q}, YPL-TKT-1, TKT-1, YPL-TKT-2, YPL-TKT-3, TKT-2, TKT-3, YPL-TKT-4, TKT-4, YPL-TKT-5 or TKT-5. ATCC13032-pXMJ19-TKT^{A327T} is recombinant bacteria which is obtained by introducing pXMJ19-TKT^{A327T} into *Corynebacterium glutamicum* ATCC13032; ATCC13032-pXMJ19-TKT^{A327S} is recombinant bacteria which is obtained by introducing pXMJ19-TKT^{A327S} into *Corynebacterium glutamicum* ATCC13032;
ATCC13032-pXMJ19-TKT^{A327C} is recombinant bacteria which is obtained by introducing pXMJ19-TKT^{A327C} into *Corynebacterium glutamicum* ATCC13032;
ATCC13032-pXMJ19-TKT^{A327P} is recombinant bacteria which is obtained by introducing pXMJ19-TKT^{A327P} into *Corynebacterium glutamicum* ATCC13032;
ATCC13032-pXMJ19-TKT^{A327N} is recombinant bacteria which is obtained by introducing pXMJ19-TKT^{A327N} into *Corynebacterium glutamicum* ATCC13032;
ATCC13032-pXMJ19-TKT^{A327Q} is recombinant bacteria which is obtained by introducing pXMJ19-TKT^{A327Q} into *Corynebacterium glutamicum* ATCC13032;
YPL-TKT-1 differs from *Corynebacterium glutamicum* YP097158 only in that YPL-TKT-1 is a strain which is obtained by replacing a gene of *Corynebacterium glutamicum* YP097158 as represented by SEQ ID No. 1 with a gene as represented by SEQ ID No. 3, and keeping other sequences unchanged; and
TKT-1 differs from *Corynebacterium glutamicum* ATCC13032 only in that TKT-1 is a strain which is obtained by replacing a gene of *Corynebacterium glutamicum* ATCC13032 as represented by SEQ ID No. 1 with a gene as represented by SEQ ID No. 3, and keeping other sequences unchanged.

YPL-TKT-2 is recombinant bacteria which is obtained by replacing a spacer region between an upper homologous arm NCgl1741 and a lower homologous arm NCgl1742 in a genome of *Corynebacterium glutamicum* YP097158 with a DNA fragment as represented by SEQ ID No. 16 in the sequence listing, and keeping other nucleotides unchanged;
YPL-TKT-3 is recombinant bacteria which is obtained by replacing the spacer region between an upper homologous arm NCgl1741 and a lower homologous arm NCgl1742 in the genome of *Corynebacterium glutamicum* YP097158 with a DNA fragment as represented by SEQ ID No. 17 in the sequence listing, and keeping other nucleotides unchanged;
TKT-2 is recombinant bacteria which is obtained by replacing a spacer region between an upper homologous arm NCgl1741 and a lower homologous arm NCgl1742 in a genome of *Corynebacterium glutamicum* ATCC13032 with a DNA fragment as represented by SEQ ID No. 16 in the sequence listing, and keeping other nucleotides unchanged;
TKT-3 is recombinant bacteria which is obtained by replacing the spacer region between the upper homologous arm NCgl1741 and the lower homologous arm NCgl1742 in the genome of *Corynebacterium glutamicum* ATCC13032 with a DNA fragment as represented by ID No. 17 in the sequence listing, and keeping other nucleotides in the genome of *Corynebacterium glutamicum* ATCC13032 unchanged;
YPL-TKT-4 is recombinant bacteria which is obtained by introducing pXMJ19-TKT into *Corynebacterium glutamicum* YP097158;
YPL-TKT-5 is recombinant bacteria which is obtained by introducing pXMJ19-TKT^{A327T} into *Corynebacterium glutamicum* YP097158;
TKT-4 is recombinant bacteria which is obtained by introducing pXMJ19-TKT into *Corynebacterium glutamicum* ATCC13032; and
TKT-5 is recombinant bacteria which is obtained by introducing pXMJ19-TKT^{A327T} into *Corynebacterium glutamicum* ATCC13032.

The present invention further provides a method for preparing L-lysine. The method includes: expressing a TKT protein in a receptor biological cell, or increasing the content or activity of the TKT protein in the receptor biological cell, or increasing the content or activity of the protein as represented by SEQ ID No. 4 or SEQ ID No. 6 or SEQ ID No. 8 or SEQ ID No. 10 or SEQ ID No. 12 or SEQ ID No. 14 or SEQ ID No. 2 in the receptor biological cell to obtain a recombinant biological cell; and culturing the recombinant biological cell to obtain the L-lysine.

In the above method, the biological cell may be yeast, bacteria, algae, fungus, a plant cell or an animal cell that can synthesize the L-lysine.

The biological cell is any biological cell that can synthesize a target amino acid.

In the above method, the bacteria is *Corynebacterium glutamicum.*

The bacteria of the present invention include, but is not limited to, *Corynebacterium glutamicum.* L-lysine may be produced from any TKT mutant protein as represented by SEQ ID No. 2, 4, 6, 8, 10, 12 or 14 and associated biomaterial of the present invention, which contain the TKT gene as represented by SEQ ID No. 1 in the sequence listing and are capable of synthesizing L-lysine. The bacteria may be *Escherichia coli, Corynebacterium glutamicum, brevibacterium lactofermentum, Corynebacterium pekinense, Brevibacterium ammoniagenes, Corynebacterium crenatum, Pantoea, Pantoea ananatis, Bacillus brevis, Lactobacillus brevis* or *Brevibacterium flavum.* The yeast may be *Saccharomyces cerevisiae* or *Pichia pastoris.*

In an example of the present invention, the *Corynebacterium glutamicum* is *Corynebacterium glutamicum* YP097158 or *Corynebacterium glutamicum* ATCC13032.

The above method may be implemented by introducing an encoding gene of the TKT protein into the receptor biological cell and expressing this encoding gene, or introducing the encoding gene of the protein as represented by SEQ ID No. 4 or SEQ ID No. 6 or SEQ ID No. 8 or SEQ ID No. 10 or SEQ ID No. 12 or SEQ ID No. 14 or SEQ ID No. 2 into the receptor biological cell and expressing this encoding gene; or
the receptor biological cell contains the DNA molecule as represented by SEQ ID No. 1, and the method is implemented by replacing a DNA molecule as represented by SEQ ID No. 1 in the receptor biological cell with a DNA molecule as represented by SEQ ID No. 3 or SEQ ID No. 5 or SEQ ID No. 7 or SEQ ID No. 9 or SEQ ID No. 11 or SEQ ID No. 13.

In the above method, the recombinant biological cell may be cultured using a medium that enables the recombinant biological cell to grow; and/or
the recombinant biological cell is cultured under conditions that enable the recombinant biological cell to grow.

The recombinant biological cell may be the recombinant microorganism described above.

The present invention further provides a product for preparing L-lysine, the product containing (or its active ingredient being)) TKT or the biomaterial.

The application of TKT or the biomaterial in the production of L-lysine, or in the preparation of a product for producing L-lysine also falls within the protection scope of the present invention.

The application of TKT or the biomaterial or the method for preparing L-lysine or the product for preparing L-lysine in the preparation of food, feed or medicine containing L-lysine also falls within the protection scope of the present invention.

TKT and the biomaterial of the present invention may be used to produce a variety of products, including but not limited to lysine in an example. The resulting products may also be glutamic acid, threonine, tryptophan, arginine, valine, glycine, alanine, leucine, isoleucine, methionine, proline, serine, tyrosine, cysteine, phenylalanine, asparagine, glutamine, aspartic acid, histidine, shikimic acid, protocatechuic acid, succinic acid, α-ketoglutarate, citric acid, ornithine, citrulline, etc.

The present invention is described in further detail below in conjunction with the specific embodiments, and the given examples are only for the purpose of clarifying the present invention, but not for the purpose of limiting the scope of the present invention. The examples provided below may be used as a guide for further improvement by a person of ordinary skill in the art and do not in any way constitute a limitation of the present invention.

### Instruction for the preservation of biomaterial

Classification and naming: *Corynebacterium glutamicum*
Strain number: YP097158
Title of preservation unit: China General Microbiological Culture Collection Center
Abbreviation of preservation unit: CGMCC
Address of preservation unit: No. 3, Courtyard 1, Beichen West Road, Chaoyang District, Beijing, Postal Code: 100101
Preservation date: August 16, 2016
Preservation Center registration number: CGMCC No. 12856

### Detailed Description of the Invention

The experimental methods used in the following examples are conventional methods unless otherwise specified. The materials, reagents, instruments, etc. used in the following examples, unless otherwise specified, may be obtained commercially. In the quantitative test in the following examples, three replicate experiments were set, and the results were averaged. In the following examples, unless otherwise specified, a first position of each nucleotide sequence in the sequence listing was a 5'-end nucleotide of corresponding DNA, and a last position was a 3'-end nucleotide of the corresponding DNA.

*Corynebacterium glutamicum* YP097158 in the following example had been preserved in the China General Microbiological Culture Collection Center (CGMCC for short, address: No. 3, Courtyard 1, Beichen West Road, Chaoyang District, Beijing, the Institute of Microbiology at the Chinese Academy of Sciences) on August 16, 2016, with the preservation number of CGMCC No. 12856. NINGXIA EPPEN NEW MATERIALS., LTD., a depositor of *Corynebacterium glutamicum* YP097158, had authorized HEILONGJIANG EPPEN NEW MATERIALS., LTD. to use this strain.

### Example 1: Construction of ATCC13032 strain containing TKT gene mutant

### I. Construction of TKT gene mutant plasmid

First, a wild type TKT gene (having a sequence as represented by SEQ ID No. 1) and its promoter were cloned into an expression vector pXMJ19. A wild type TKT promoter and an encoding region fragment of 2689 bp (having a sequence as represented by SEQ ID No. 15) were obtained by taking a genome sequence of *Corynebacterium glutamicum* ATCC13032 published by NCBI as a template and performing PCR amplification by using primers TKT-F/TKT-R. The recovered fragment was linked to an expression vector pXMJ19 (TaKaRa, having chloramphenicol resistance) recovered by Xbal I and BamH I enzyme digestion through an NEBuilder enzyme (NEB) at 50°C for 30 min; the linking product was transformed to DH5α competent cells, coated onto a 2-YT agar plate containing chloramphenicol (34 mg/L), and cultured at 37°C for 12 h; and monoclones which grew in culture were subjected to PCR identification by using primers TKT-F/TKT-R. The ones that could produce a fragment size of 2689 bp through PCR amplification were a positive transformant pXMJ19-TKT which contained a TKT promoter and an encoding region sequence.

In order to obtain a mutant that encoded the TKT gene, a random mutagenesis kit (Agilent Technologies, USA) was used to prepare a TKT mutant gene plasmid. By taking the constructed plasmid pXMJ19-TKT as a template, PCR amplification was performed by using the primers TKT-F/TKT-R to obtain a TKT gene encoding region and promoter region fragment (having a sequence as represented by SEQ ID No. 15, but there were random point mutations in the TKT encoding region) containing random point mutations.

The recovered DNA fragment was linked to an expression vector pXMJ19 (TaKaRa, containing chloramphenicol resistance) recovered by Xbal I and BamH I enzyme digestion through an NEBuilder enzyme (NEB) at 50°C for 30 min; and the linking product was transformed to DH5α, coated onto a 2-YT agar plate containing chloramphenicol (34 mg/L), and cultured at 37°C for 12 h. The monoclones which grew in culture were subjected to PCR identification by using primers TKT-F/TKT-R. The ones which could produce a fragment size of 2689 bp (having a sequence as represented by SEQ ID No. 15, but there were random point mutations in a TKT encoding region) were a pXMJ19-TKT-MT positive transformant which contained TKT gene random mutations.

Positions 35-437 in SEQ ID No. 15 were shown as a TKT gene promoter.

The primer design was as follows (synthesized by Shanghai Invitrogen Company):
TKT-F: 5'-CAGAATAATTAAGCTTGCATGCCTGCAGGTCGACTTCACAGCGGACGATTTC-3' (the underlined nucleotide sequence was a pXMJ19 homologous sequence, SEQ ID No. 18), and
TKT-R: 5'-CCAAAACAGCCAAGCTGAATTCGAGCTCGGTACCAGGCAAGTAAGGGATGTGC-3' (the underlined nucleotide sequence was a pXMJ19 homologous sequence, SEQ ID No. 19).

### II. Construction of strain containing mutant TKT gene plasmid

In order to identify the production performance of the mutant vector pXMJ19-TKT-MT constructed in step I in L-amino acid, specifically, different TKT random mutant plasmids constructed in step I were transformed into a wild type *Corynebacterium glutamicum* ATCC13032 strain by electric shock (see WO2014121669A1 for a specific transformation method), and subjected to PCR identification by using primers TKT-F/TKT-R. The ones which could produce a fragment size of 2689 bp through PCR amplification were a positive transformant. The positive transformant was consecutively passaged three times on a culture plate (see Table 1 for medium components and culture conditions) containing chloramphenicol (34 mg/L), then inoculated into a 500 mL triangular flask filled with 30 mL of rich medium and fermented at 30°C for 48 h under shaking. The concentration of L-amino acid was detected by high-performance liquid chromatography (HPLC) after fermentation culture. As shown in Table 2, each strain, i.e., a ATCC13032-pXMJ19-TKT mutant strain, with superior L-amino acid production ability compared to the wild type *Corynebacterium glutamicum* ATCC13032 control was picked.

Rich medium: water was used as a solvent; solute and its concentration were 30 g/L glucose, 2 g/L (NH4)₂SO₄, 0.5 g/L H₃PO₄, 0.8 g/L KCl, 0.8 g/L MgSO₄ . 7H₂O, 0.05 g/L FeSO₄ . 7H₂O, 0.05 g/L MnSO₄ . H₂O, 1.5 g/L FM902 yeast powder, 1.5 g/L corn syrup, 17 g/L molasses, 0.5 g/L betaine, 2 g/L citric acid, 20 mg/L VH, 1.5 mg/L VB₁, 1.5 mg/L VB₃, 1.5 g/L VB₁₂, and sodium hydroxide (adjusted to pH7.0).

**Table 1: Compositions and culture conditions of mediums**

| Components | Formula |
|---|---|
| Sucrose | 10 g/L |
| Polypeptone | 10 g/L |
| Beef extract | 10 g/L |
| Yeast powder | 5 g/L |
| Urea | 2 g/L |
| Sodium chloride | 2.5 g/L |
| Agar powder | 18 g/L |
| pH | 7.0 |
| Culture temperature | 32°C |
| Culture time | 12 h |

**Table 2: High-performance liquid chromatography analysis results of ATCC13032-pXMJ19-TKT mutant strains**

| Names of L-amino acids | Content of L-amino acids (g/100mL) | | | | | |
|---|---|---|---|---|---|---|
| | ATCG130 32 | ATCC13032-TKT mutant strain 1 | ATGC13032-TKT mutant strain 2 | ATCC13032-TKT mutant strain 3 | ATCC13032-TKT mutant strain 4 | ATCG13032-TKT mutant strain 5 |
| L-aspartic acid | Not detected | Not detected | Not detected | Not detected | Not detected | Not detected |
| L-glutamic | 0.005 | 0.004 | 0.007 | 0.003 | 0.003 | 0.001 |
| L-serine | Not detected | Not detected | Not detected | Not detected | Not detected | Not detected |
| L-arginine | 0.004 | 0.001 | 0.001 | 0.007 | 0.014 | 0.021 |
| L-glycine | Not detected | Not detected | Not detected | Not detected | Not detected | Not detected |
| L-threonine | 0.008 | Not detected | 0.001 | Not detected | 0.001 | 0.015 |
| L-lysine | 0.004 | 0.161 | 0.078 | 0.597 | 0.045 | 0.014 |
| L-proline | Not detected | Not detected | Not detected | Not detected | Not detected | Not detected |
| L-alanine | Not detected | Not detected | Not detected | Not detected | Not detected | Not detected |
| L-valine | 0.006 | 0.005 | 0.005 | 0.006 | Not detected | Not detected |
| L-methionin | 0.001 | Not detected | Not detected | Not detected | Not detected | Not detected |
| L-cysteine | Not detected | Not detected | Not detected | Not detected | Not detected | Not detected |
| L-isoleucine | Not detected | Not detected | 0.002 | Not detected | Not detected | Not detected |
| L-leucine | Not detected | Not detected | Not detected | Not detected | Not detected | Not detected |
| L-phenylala nine | Not *detected* | Not detected | Not detected | Not detected | Not detected | Not detected |
| L-tyrosine | *Not detected* | Not detected | Not detected | Not detected | Not detected | Not detected |

As shown in Table 2, the *Corynebacterium glutamicum* ATCC13032-pXMJ19-TKT mutant strain had the ability to produce some L-lysine, wherein the ATCC13032-pXMJ19-TKT mutant strain 3 had a superior ability to produce L-lysine, indicating that the TKT gene mutant strain 3 had the activity of synthesizing L-lysine.

A plasmid was extracted from the ATCC13032-pXMJ19-TKT mutant strain 3, and the TKT gene was sequenced. The results proved that guanine (G) at position 979 in a nucleotide sequence of an encoding region of the TKT gene was mutated into adenine (A) (having a sequence as represented by SEQ ID No. 3, a gene containing this mutation being denoted as a TKT^{A327T} gene); alanine (A) at position 327 in a corresponding amino acid sequence was mutated into threonine (T) (having a sequence as represented by SEQ ID No. 4, a protein containing this mutation being denoted as a TKT^{A327T} protein); and this plasmid was denoted as pXMJ19-TKT^{A327T}, and a mutant strain containing this plasmid was renamed as ATCC13032-pXMJ19-TKT^{A327T}.

pXMJ19-TKT^{A327T} differed from pXMJ19-TKT in that: pXMJ19-TKT^{A327T} was a recombinant vector which was obtained by replacing a wild type TKT gene in pXMJ19-TKT with a mutant TKT^{A327T} gene sequence, and pXMJ19-TKT^{A327T} was capable of expressing a TKT^{A327T} mutant protein. The wild type TKT gene differed from the mutant TKT^{A327T} gene only in that: GCT was located at positions 979-981 of the wild type TKT gene, and ACT was located at positions 979-981 of the mutant TKT^{A327T} gene. The wild type TKT protein differed from the TKT^{A327T} mutant protein only in that: an alanine residue A was located at position 327 of the wild type TKT protein, and a threonine residue T was located at position 327 of the TKT^{A327T} mutant protein.

### III. Construction of TKT gene mutant strains and recombinant vector

A mutant strain ATCC13032-pXMJ19-TKT^{A327T} was obtained using wild type *Corynebacterium glutamicum* ATCC13032 by means of random mutation. In order to obtain more TKT mutant strains to improve their L-lysine production abilities, a mutant with the same mutation site as the aforementioned TKT but with a different amino acid therefrom was constructed. Specifically, by taking the plasmid pXMJ19-TKT^{A327T} sequenced in step II as a template, five mutants were constructed in which amino acid at position 327 of TKT was replaced by different amino acids. The replaced amino acids were all hydrophilic amino acids. The names of the replaced amino acids in the mutants and primers used in the respective mutants were shown in Table 3.

**Table 3: Names of the replaced amino acids in the TKT mutants and primers used in the respective mutants**

| Genes | Replaced amino acids | Primers | Names of pXMJ19-TKT mutant vectors |
|---|---|---|---|
| TKT | A327S | TKT-F/S-PR-1, S-PR-2/TKT-R | pXMJ19-TKT^{A327S} |
| | A327C | TKT-F/C-PR-1, C-PR-2/TKT-R | pXMJ19-TKT^{A327S} |
| | A327P | TKT-F/P-PR-1, P-PR-2/TKT-R | pXMJ19-TKT^{A327S} |
| | A327N | TKT-F/N-PR-1, N-PR-2/TKT-R | pXMJ19-TKT^{A327N} |
| | A327Q | TKT-F/Q-PR-1, Q-PR⁻2/TKT⁻R | pXMJ19-TKT^{A327Q} |

The primer design was as follows (synthesized by Shanghai Invitrogen Company):
S-PR-1: 5'-CCATGCAGCCTTCTTCTGTGCAGAGCGCTCTG-3' (SEQ ID No. 20),
S-PR-2: 5'-CAGAGCGCTCTGCACAGAAGAAGGCTGCATGG-3' (SEQ ID No. 21),
C-PR-1: 5'-CCATGCAGCCTTCTTCTGTGCACAGCGCTCTG-3' (SEQ ID No. 22),
C-PR-2:5'-CAGAGCGCTGTGCACAGAAGAAGGCTGCATGG-3'(SEQ ID No. 23),
P-PR-1: 5'-CCATGCAGCCTTCTTCTGTGCAGGGCGCTCTG-3' (SEQ ID No. 24),
P-PR-2: 5'-CAGAGCGCCCTGCACAGAAGAAGGCTGCATGG-3' (SEQ ID No. 25),
N-PR-1: 5'-CCATGCAGCCTTCTTCTGTGCATTGCGCTCTG-3' (SEQ ID No. 26),
N-PR-2: 5'-CAGAGCGCAATGCACAGAAGAAGGCTGCATGG-3' (SEQ ID No. 27),
Q-PR-1: 5'-CCATGCAGCCTTCTTCTGTGCCTGGCGCTCTG-3' (SEQ ID No. 28),
Q-PR-2: 5'-CAGAGCGCCAGGCACAGAAGAAGGCTGCATGG-3' (SEQ ID No. 29).

By taking a wild type *Corynebacterium glutamicum* ATCC13032 genome as a template, PCR amplification was performed by using primers TKT-F/S-PR-1 and KAPA HiFi HotStart in Table 2 to obtain an Up DNA fragment (1405 bp) with a TKT mutant base. PCR amplification was performed by using primers S-PR-2/TKT-R and KAPA HiFi HotStart to obtain a Down DNA fragment (1248 bp) with a TKT mutant base. After the PCR reaction, agarose gel electrophoresis recovery was performed by a column DNA gel recovery kit, respectively. The recovered DNA fragment was linked to an expression vector pXMJ19 recovered by Xbal I and BamH I enzyme digestion through an NEBuilder enzyme (NEB) at 50°C for 30 min; and the linking product was transformed to DH5α, coated onto a 2-YT agar plate containing chloramphenicol (34 mg/L), and cultured at 37°C for 12 h. Monoclones which grew in culture were subjected to PCR identification by using primers TKT-F/TKT-R, and the ones that could produce a fragment of 2689 bp were a positive transformant ATCC13032-pXMJ19-TKT^{A327S} in which alanine at position 327 of a protein that encoded the TKT gene was mutated into tryptophan. The other four strains were constructed in the same way: five pXMJ19-TKT mutant vectors and strains with alanine at position 327 replaced by each of the amino acids in Table 3 were named according to the names listed in Table 3. The resulting mutant strains were ATCC13032-pXMJ19-TKT^{A327C}, ATCC13032-pXMJ19-TKT^{A327P}, ATCC13032-pXMJ19-TKT^{A327N}, and ATCC13032-pXMJ19-TKT^{A327Q}.

ATCC13032-pXMJ19-TKT^{A327S} contained a recombinant vector pXMJ19-TKT^{A327S}. pXMJ19-TKT^{A327S} differed from pXMJ19-TKT in that: pXMJ19-TKT^{A327S} was a recombinant vector which was obtained by replacing a wild type TKT gene in pXMJ19-TKT with a mutant TKT^{A327S} gene sequence (as represented by SEQ ID No. 5), and pXMJ19-TKT^{A327S} was capable of expressing a TKT^{A327S} mutant protein (as represented by SEQ ID No. 6). The wild type TKT gene differed from the mutant TKT^{A327S} gene only in that: GCT was located at positions 979-981 of the wild type TKT gene, and TCT was located at positions 979-981 of the mutant TKT^{A327S} gene. The wild type TKT protein differed from the TKT^{A327S} mutant protein only in that: an alanine residue A was located at position 327 of the wild type TKT protein, and a serine residue S was located at position 327 of the TKT^{A327S} mutant protein.

ATCC13032-pXMJ19-TKT^{A327C} contained a recombinant vector pXMJ19-TKT^{A327C}. pXMJ19-TKT^{A327C} differed from pXMJ19-TKT in that: pXMJ19-TKT^{A327C} was a recombinant vector which was obtained by replacing a wild type TKT gene in pXMJ19-TKT with a mutant TKT^{A327C} gene sequence (as represented by SEQ ID No. 7), and pXMJ19-TKT^{A327C} was capable of expressing a TKT^{A327C} mutant protein (as represented by SEQ ID No. 8). The wild type TKT gene differed from the mutant TKT^{A327C} gene only in that: GCT was located at positions 979-981 of the wild type TKT gene, and TCT was located at positions 979-981 of the mutant TKT^{A327C} gene. The wild type TKT protein differed from the TKT^{A327C} mutant protein only in that: an alanine residue A was located at position 327 of the wild type TKT protein, and a cysteine residue C was located at position 327 of the TKT^{A327C} mutant protein.

ATCC13032-pXMJ19-TKT^{A327P} contained a recombinant vector pXMJ19-TKT^{A327P}. pXMJ19-TKT^{A327P} differed from pXMJ19-TKT in that: pXMJ19-TKT^{A327P} was a recombinant vector which was obtained by replacing a wild type TKT gene in pXMJ19-TKT with a mutant TKT^{A327P} gene sequence (as represented by SEQ ID No. 9), and pXMJ19-TKT^{A327P} was capable of expressing a TKT^{A327P} mutant protein (as represented by SEQ ID No. 10). The wild type TKT gene differed from the mutant TKT^{A327P} gene only in that: GCT was located at positions 979-981 of the wild type TKT gene, and CCT was located at positions 979-981 of the mutant TKT^{A327P} gene. The wild type TKT protein differed from the TKT^{A327P} mutant protein only in that: an alanine residue A was located at position 327 of the wild type TKT protein, and a proline residue P was located at position 327 of the TKT^{A327P} mutant protein.

ATCC13032-pXMJ19-TKT^{A327N} contained a recombinant vector pXMJ19-TKT^{A327N}. pXMJ19-TKT^{A327N} differed from pXMJ19-TKT in that: pXMJ19-TKT^{A327N} was a recombinant vector which was obtained by replacing a wild type TKT gene in pXMJ19-TKT with a mutant TKT^{A327N} gene sequence (as represented by SEQ ID No. 11), and pXMJ19-TKT^{A327N}was capable of expressing a TKT^{A327N} mutant protein (as represented by SEQ ID No. 12). The wild type TKT gene differed from the mutant TKT^{A327N} gene only in that: GCT was located at positions 979-981 of the wild type TKT gene, and AAT was located at positions 979-981 of the mutant TKT^{A327N} gene. The wild type TKT protein differed from the TKT^{A327N} mutant protein only in that: an alanine residue A was located at position 327 of the wild type TKT protein, and an asparagine residue N was located at position 327 of the TKT^{A327N} mutant protein.

ATCC13032-pXMJ19-TKT^{A327Q} contained a recombinant vector pXMJ19-TKT^{A327Q}. pXMJ19-TKT^{A327Q} differed from pXMJ19-TKT in that: pXMJ19-TKT^{A327Q} was a recombinant vector which was obtained by replacing a wild type TKT gene in pXMJ19-TKT with a mutant TKT^{A327Q} gene sequence (as represented by SEQ ID No. 13), and pXMJ19-TKT^{A327Q} was capable of expressing a TKT^{A327Q} mutant protein (as represented by SEQ ID No. 14). The wild type TKT gene differed from the mutant TKT^{A327Q} gene only in that: GCT was located at positions 979-981 of the wild type TKT gene, and CAG was located at positions 979-981 of the mutant TKT^{A327Q} gene. The wild type TKT protein differed from the TKT^{A327Q} mutant protein only in that: an alanine residue A was located at position 327 of the wild type TKT protein, and a glutamine residue Q was located at position 327 of the TKT^{A327Q} mutant protein.

### IV. Detection of L-lysine production ability of TKT mutant strains

In order to identify the production performance of the mutant vector constructed in step III against L-lysine, specifically, five recombinant bacteria constructed in step III and ATCC13032-pXMJ19-TKT^{A327T} in step II were consecutively passaged three times on a culture plate containing chloramphenicol (34 mg/L), then inoculated into a 500 mL triangular flask filled with 30 mL of rich medium and fermented at 37°C for 48 h under shaking. After the fermentation culture, the concentration of L-lysine was analyzed by high-performance liquid chromatography (HPLC), and the wild type *corynebacterium glutamicum* ATCC13032 was used as a control.

The results were shown in Table 4. The L-lysine yield of each mutant strain was significantly higher than that of the wild type *Corynebacterium glutamicum* ATCC13032, while the ability of the mutant strain ATCC13032-pXMJ19-TKT^{A327T} to produce L-lysine was superior to that of ATCC13032-pXMJ19-TKT^{A327S}, ATCC13032-pXMJ19-TKT^{A327C}, ATCC13032-pXMJ19-TKT^{A327P}, ATCC13032-pXMJ19-TKT^{A327N} and ATCC13032-pXMJ19-TKT^{A327Q}, indicating that alanine (A) at position 327 of the TKT protein was mutated into threonine (T), which was more conducive to lysine accumulation.

**Table 4: L-tyrosine detection results of W3110-TKT mutant strains by high-performance liquid chromatography**

| Strains | L-lysine (g/100mL) |
|---|---|
| ATCC13032 | 0.002 |
| ATCC13032-pXMJ19-TKT^{A327T} | 0.585 |
| ATCC13032-pXMJ19-TKT^{A327S} | 0.138 |
| ATCC13032-pXMJ19-tkt^{A327C} | 0.076 |
| ATCC13032-pXMJ19-TKT^{A327P} | 0.084 |
| ATCC13032-pXMJ19-TKT^{A327N} | 0.113 |
| ATCC13032-pXMJ19-TKT^{A327}Q | 0.071 |

### Example 2: Construction of recombinant vector having TKT gene encoding region containing point mutations

According to a genomic sequence of *Corynebacterium glutamicum* ATCC13032 published by NCBI, two pairs of primers that amplify a TKT gene encoding region were designed and synthesized. Point mutation was introduced into a TKT gene encoding region (SEQ ID No. 1) of *Corynebacterium glutamicum* YP097158 (preservation number: CGMCC No. 12856, preservation date: August 16, 2016, preservation unit: the Institute of Microbiology at Chinese Academy of Sciences, Beichen West Road, Chaoyang District, Beijing, Tel: 010-64807355) by using an allelic substitution method. The point mutation was to mutate guanine (G) at position 979 in the nucleotide sequence (SEQ ID No. 1) of the TKT gene into adenine (A) to obtain a DNA molecule as represented by SEQ ID No. 3 (a mutated TKT gene, named as a TKT^{A327T} gene).

The DNA molecule as represented by SEQ ID No. 1 encoded a protein having an amino acid sequence as represented by SEQ ID No. 2. The DNA molecule (TKT^{A327T} gene) as represented by SEQ ID No. 3 encoded a mutant protein (the mutant protein being name as a TKT^{A327T} protein) having an amino acid sequence as represented by SEQ ID No. 4. Threonine (T) at position 327 in the amino acid sequence (SEQ ID No. 4) of the mutant protein TKT^{A327T} was mutated from alanine (A).

A recombinant vector was constructed by using the NEBuilder recombination technology, and the primers were designed as follows (synthesized by Shanghai Invitrogen Company). The bolded bases represented mutation sites:
P1: 5'-CAGTGCCAAGCTTGCATGCCTGCAGGTCGACTCTAGCGTCATTGCTTCTGATGG-3' (the underlined nucleotide sequence was a sequence on pK18, SEQ ID No. 30),
P2: 5'-CCATGCAGCCTTCTTCTGTGCAGTGCGCTCTG-3' (SEQ ID No. 31),
P3: 5'-CAGAGCGCACTGCACAGAAGAAGGCTGCATGG-3' (SEQ ID No. 32),
P4: 5'-CAGCTATGACCATGATTACGAATTCGAGCTCGGTACCCCGGAGAAGATGAGGAAGGTT C-3' (the underlined nucleotide sequence was a sequence on pK18, SEQ ID No. 33).

Construction method: by taking *Corynebacterium glutamicum* ATCC13032 as a template, PCR amplification was performed by using primers P1 and P2, P3 and P4, respectively, to obtain two DNA fragments (TKTUp and TKTDown) of the TKT gene encoding region, which carried mutant bases and had the sizes of 514 bp and 482 bp respectively.

The two DNA fragments (TKTUp and TKTDown) were separated and purified by agarose gel electrophoresis, and then linked to a pK18mobsacB plasmid (Addgene) purified by enzyme digestion (Xbal I/BamH I) through an NEBuilder enzyme (NEB) at 50°C for 30 min. Monoclones which grew after transformation of the linking product were subjected to PCR identification by using primers P1/P4. The ones which could produce a fragment of 964 bp were a positive recombinant vector pK18-TKT^{A327T}. This recombinant vector contained a kanamycin resistance marker. The correctly enzymatically digested recombinant vector pK18-TKT^{A327T} was delivered to the sequencing company for sequencing and verification. A recombinant vector pK18-TKT^{A327T} containing a correct point mutation (G979A) was preserved for future use.

This recombinant vector pK18-TKT^{A327T} contained a mutation site (G979A), which will cause guanine (G) at position 979 in a TKT gene (SEQ ID No. 1) encoding region of the strain *Corynebacterium glutamicum* YP097158 to be mutated into adenine (A), ultimately resulting in the change of alanine (A) at position 327 of the encoding protein to threonine (T).

The recombinant vector pK18- TKT^{A327T} was a recombinant vector which was obtained by replacing a fragment (small fragment) between Xbal I and BamH I recognition sites of a pK18mobsacB vector with a DNA fragment as represented by SEQ ID No. 6 in the sequence listing, and keeping other sequences of the pK18mobsacB vector unchanged. The recombinant vector pK18-TKT^{A327T} contained a mutation site (G979A) of the mutant gene TKT^{A327T} as represented by SEQ ID No. 3.

### Example 3: Construction of engineered strain containing gene TKT^{A327T}

The allelic substitution plasmid (pK18-TKT^{A327T}) constructed in Example 2 was transformed by electric shock into a strain *Corynebacterium glutamicum* YP097158 (after sequencing, it was confirmed that a wild type TKT gene encoding region was retained on chromosomes of this strain) and a wild type strain *Corynebacterium glutamicum* ATCC13032, and cultured on a solid culture plate containing kanamycin (50 mg/L) for 40 h. Single colonies produced in culture were identified by using primers P1/P4 in Example 2, respectively. A strain that could amplify a stripe of 964 bp was a positive strain. The positive strain was subjected to streak culture on a medium containing 15% of sucrose (this medium was obtained by increasing the sucrose concentration in the medium in Table 1 to 150 g/L). The single colonies produced in culture were cultured on a kanamycin-containing medium and a kanamycin-free medium, respectively. Strains that grew on the kanamycin-free medium, but did not grow on the kanamycin-containing medium were further subjected to PCR amplification using primers P1/P4. The plurality of resulting DNA fragments (964 bp) were sequenced. Through sequence alignment, the strains with a base sequence mutation (G979A) were positive strains that had successfully undergone allelic substitution. The positive strains obtained from the *Corynebacterium glutamicum* YP097158 and the wild type strain *Corynebacterium glutamicum* ATCC13032 were named as YPL-TKT-1 and TKT-1, respectively. Both the recombinant bacteria YPL-TKT-1 and TKT-1 contained a mutated gene TKT^{A327T} as represented by SEQ ID No. 3 and could express the TKT^{A327T} protein as represented by SEQ ID No. 4. The recombinant bacteria YPL-TKT-1 differed from *Corynebacterium glutamicum* YP097158 only in that: YPL-TKT-1 was a strain which was obtained by replacing a TKT gene of *Corynebacterium glutamicum* YP097158 with a TKT^{A327T} gene, and keeping other sequences unchanged. The recombinant bacteria TKT-1 differed from wild type *Corynebacterium glutamicum* ATCC13032 only in that: TKT-1 was a strain which was obtained by replacing a TKT gene of wild type *Corynebacterium glutamicum* ATCC13032 with a TKT^{A327T} gene, and keeping other sequences unchanged. Recombinant bacteria containing the mutated TKT^{A327T} gene could significantly and stably increase the expression quantity of the TKT^{A327T}gene.

### Example 4: Construction of engineered strains overexpressing TKT gene or TKT^{A327T} gene on genome

According to a genomic sequence of *Corynebacterium glutamicum* ATCC13032 published by NCBI, three pairs of primers for amplifying upstream and downstream homologous arm fragments, as well as a TKT or TKT^{A327T} gene encoding region and a promoter region were designed and synthesized. Copies of the TKT or TKT^{A327T} gene were inserted into *Corynebacterium glutamicum* YP097158 and wild type *Corynebacterium glutamicum* ATCC13032 by means of homologous recombination.

The primer design was as follows (synthesized by Shanghai Invitrogen Company):
P5: 5'-CAGTGCCAAGCTTGCATGCCTGCAGGTCGACTCTAGAATGCGTTCTGGACTGAGG-3' (the underlined nucleotide sequence was a sequence on pK18, SEQ ID No. 34),
P6: 5'-GAAATCGTCCGCTGTGAAGTGCACCGAGAACAGATG-3' (SEQ ID No. 35),
P7: 5'-CATCTGTTCTCGGTGCACTTCACAGCGGACGATTTC-3' (SEQ ID No. 36),
P8: 5'-GATTTAATTGCGCCATCTGAGGCAAGTAAGGGATGTGC-3' (SEQ ID No. 37), P9: 5'-GCACATCCCTTACTTGCCTCAGATGGCGCAATTAAATC-3' (SEQ ID No. 38), and
P10: 5'-CAGCTATGACCATGATTACGAATTCGAGCTCGGTACCCGCTATGACACCTTCAACGG ATC-3' (the underlined nucleotide sequence was a sequence on pK18, SEQ ID No. 39).

Construction method: by taking a genome of *Corynebacterium glutamicum* ATCC13032 as a template, PCR amplification was performed by using primers P5/P6, P7/P8 and P9/P10, respectively, to obtain an upstream homologous arm fragment of 763 bp (corresponding to a partial encoding region of NCgl1740 and a NCgl1741 gene and its promoter region of *Corynebacterium glutamicum* ATCC13032), a TKT gene and its promoter fragment of 2621 bp (having a sequence as represented by SEQ ID No. 16), and a downstream homologous arm fragment of 596 bp (corresponding to a partial encoding region of NCgl1742 gene of *Corynebacterium glutamicum* ATCC13032). After the PCR reaction, three fragments amplified from each template were recovered by electrophoresis using a column DNA gel recovery kit. Three recovered fragments were linked to a pK18mobsacB plasmid (Addgene), which was purified after Xbal I and BamH I enzyme digestion, at 50°C for 30 min by using a NEBuilder enzyme (NEB). Monoclones which grew after the transformation of the linking product were subjected to PCR identification by using primers M13F/M13R to obtain a positive integrated plasmid (recombinant vector). The resulting recombinant vector was pK18-TKTOE. This positive integrated plasmid contained a kanamycin resistance marker, and a recombinant in which a plasmid was integrated into a genome was obtained by kanamycin screening. In SEQ ID No.16, positions 1-403 were promoters of the TKT gene, and positions 404-2506 were the TKT gene.
M13F: 5'-TGTAAAACGACGGCCAGT-3' (SEQ ID No. 40),
M13R: 5'-CAGGAAACAGCTATGACC-3' (SEQ ID No. 41).

By taking a genome of *Corynebacterium glutamicum* YPL-TKT-1 as a template, PCR amplification was performed by using primers P5/P6, P7/P8 and P9/P10, respectively, to obtain an upstream homologous arm fragment of 763 bp (corresponding to a partial encoding region of NCgl1740 and a NCgl1741 gene and its promoter region of *Corynebacterium glutamicum* ATCC13032), a TKT^{A327T} gene and its promoter fragment of 2621 bp (having a sequence as represented by SEQ ID No. 17), and a downstream homologous arm fragment of 596 bp (corresponding to a partial encoding region of NCgl1742 gene of *Corynebacterium glutamicum* ATCC13032). After the PCR reaction, three fragments amplified from each template were recovered by electrophoresis using a column DNA gel recovery kit. Three recovered fragments were linked to a pK18mobsacB plasmid (Addgene), which was purified after Xbal I and BamH I enzyme digestion, at 50°C for 30 min by using a NEBuilder enzyme (NEB). Monoclones which grew after the transformation of the linking product were subjected to PCR identification by using primers M13F/M13R to obtain a positive integrated plasmid (recombinant vector). The resulting recombinant vector was pK18-TKT ^{A327T}OE. This positive integrated plasmid contained a kanamycin resistance marker, and a recombinant in which a plasmid was integrated into a genome was obtained by kanamycin screening. In SEQ ID No. 17, positions 1-403 were promoters of the TKT^{A327T} gene, and positions 404-2506 were the TKT^{A327T} gene.

The correctly sequenced integrated plasmids (pK18-TKTOE, pK18-TKT^{A327T}OE) were respectively electrotransformed into the *Corynebacterium glutamicum* YP097158 and wild type *Corynebacterium glutamicum* ATCC13032, and cultured in a medium for 30 h. Single colonies which were produced in culture were subjected to PCR identification by using primers P11/P12, and the ones which could produce a fragment of 1559 bp through PCR amplification were positive strains, while those that did not produce any fragment were probiotics. The positive strains were subjected to streak culture on a solid culture plate containing 15% of sucrose for 30 h. Single colonies which were produced in culture were further subjected to PCR identification by using primers P13/P14. The bacteria which had a size of 1559 bp through amplification was a positive strain in which a TKT or TKT^{A327T} gene and its promoter were integrated into a spacer region between an upper homologous arm NCgl1741 and a lower homologous arm NCgl1742 on a genome of *Corynebacterium glutamicum.* The strains obtained from *Corynebacterium glutamicum* YP097158 as starting bacteria were named as YPL-TKT-2 (without mutation points) and YPL-TKT-3 (with mutation points), respectively. The strains obtained from *Corynebacterium glutamicum* ATCC13032 as starting bacteria were named as TKT-2 (without mutation points) and TKT-3 (with mutation points), respectively.

The recombinant bacteria YPL-TKT-2 contained dual copies of the TKT gene as represented by SEQ ID No. 1. Specifically, the recombinant bacteria YPL-TKT-2 was recombinant bacteria which was obtained by replacing a spacer region between an upper homologous arm NCgl1741 and a lower homologous arm NCgl 1742 in a genome of *Corynebacterium glutamicum* YP097158 with a DNA fragment as represented by SEQ ID No. 16 (positions 1-403 of SEQ ID No. 16 were shown as promoters and positions 404-2506 were shown as the TKT gene), and keeping other nucleotides in the genome of *Corynebacterium glutamicum* YP097158 unchanged. Recombinant bacteria containing dual copies of the TKT gene could significantly and stably increase the expression quantity of the TKT gene.

The recombinant bacteria TKT-2 contained dual copies of the TKT gene as represented by SEQ ID No. 1. Specifically, the recombinant bacterium TKT-2 was recombinant bacteria which was obtained by replacing a spacer region between an upper homologous arm NCgl1741 and a lower homologous arm NCgl 1742 in a genome of *Corynebacterium glutamicum* ATCC13032 with a DNA fragment as represented by SEQ ID No. 16, and keeping other nucleotides in the genome of *Corynebacterium glutamicum* ATCC13032 unchanged. Recombinant bacteria containing dual copies of the TKT gene could significantly and stably increase the expression quantity of the TKT gene.

The recombinant bacteria YPL-TKT-3 contained the TKT^{A327T} gene as represented by SEQ ID No. 3. Specifically, the recombinant bacteria YPL-TKT-3 was recombinant bacteria which was obtained by replacing the spacer region between the upper homologous arm NCgl1741 and the lower homologous arm NCgl1742 in the genome of *Corynebacterium glutamicum* YP097158 with a DNA fragment as represented by SEQ ID No. 17 (positions 1-403 of SEQ ID No. 17 were shown as promoters and positions 404-2506 were shown as the TKT^{A327T} gene), and keeping other nucleotides in the genome of *Corynebacterium glutamicum* YP097158 unchanged.

The recombinant bacteria TKT-3 contained the mutated TKT^{A327T} gene as represented by SEQ ID No. 3. Specifically, the recombinant bacteria TKT-3 was recombinant bacteria which was obtained by replacing a spacer region between the upper homologous arm NCgl1741 and the lower homologous arm NCgl1742 in the genome of *Corynebacterium glutamicum* ATCC13032 with a DNA fragment as represented by SEQ ID No. 17, and keeping other nucleotides in the genome of *Corynebacterium glutamicum* ATCC13032 unchanged.

The primers for PCR identification were as follows:
P11: 5'-TCCAAGGAAGATACACGCC-3' (corresponding to the outside of the upper homologous arm NCgl1740, SEQ ID No. 42),
P12: 5'-GCCACAAGAAAGAAGAGAAG-3' (corresponding to the inside of the TKT gene, SEQ ID No. 43),
P13: 5'-CATCCTCAACGGCATTTC-3' (corresponding to the inside of the TKT gene, SEQ ID No. 44), and
P14: 5'-TGGTCGTTGGAATCTTGC-3' (corresponding to the outside of the lower homologous arm NCgl1742, SEQ ID No. 45).

### Example 5: Construction of engineered strains overexpressing TKT gene or TKT^{A327T} gene on plasmids

The plasmids pXMJ19-TKT and pXMJ19-TKT^{A327T} which were successfully constructed in Example 1 were respectively electrotransformed into the *Corynebacterium glutamicum* YP097158 and wild type *Corynebacterium glutamicum* ATCC13032, and cultured in a medium (see Table 1 for medium components) for 30 h. Single colonies which were produced in culture were subjected to PCR identification using primers TKT- F/TKT- R, and the ones which could produce a fragment of 2689 bp (having a sequence as represented by SEQ ID No. 15) through PCR amplification were positive strains. The strains obtained from *Corynebacterium glutamicum* YP097158 as starting bacteria were named as YPL-TKT-4 (containing a plasmid pXMJ19-TKT) and YPL-TKT-5 (containing a plasmid pXMJ19-TKT ^{A327T}), respectively. The strains obtained from *Corynebacterium glutamicum* ATCC13032 as starting bacteria were named as TKT-4 (containing a plasmid pXMJ19-TKT) and TKT-5 (containing a plasmid pXMJ19-TKT^{A327T}), respectively.

Recombinant bacteria YPL-TKT-4 and TKT-4 containing the plasmid of the TKT gene as represented by SEQ ID No. 1 could significantly and stably increase the expression quantity of the TKT gene.

Recombinant bacteria YPL-TKT-5 and TKT-5 containing the plasmid of the TKT^{A327T} gene as represented by SEQ ID No. 3 could significantly and stably increase the expression quantity of the TKT gene.

### Example 6: Construction of engineered strain with TKT gene deficient in genome

According to a genomic sequence of *Corynebacterium glutamicum* ATCC13032 published by NCBI, two pairs of primers that amplified the fragments at both ends of a TKT gene encoding region were synthesized as upstream and downstream homologous arm fragments. The primer design was as follows (synthesized by Shanghai Invitrogen Company):
P15: 5'-CAGTGCCAAGCTTGCATGCCTGCAGGTCGACTCTAGGGAAATAGATGGGTGTAGACG-3 ' (the underlined nucleotide sequence was a sequence on pK18, SEQ ID No. 46),
P16: 5'-GCAAGGAACGGAAACAACGCCTTCAGGTCATCCATCTC-3' (SEQ ID No. 47), P17: 5'-GAGATGGATGACCTGAAGGCGTTGTTTCCGTTCCTTGC-3' (SEQ ID No. 48), and
P18: 5'-CAGCTATGACCATGATTACGAATTCGAGCTCGGTACCCATCAACCTTTGCCCACAG-3' (the underlined nucleotide sequence was a sequence on pK18, SEQ ID No. 49).

Construction method: by taking the genome of *Corynebacterium glutamicum* ATCC13032 as a template, PCR amplification was performed by using primers P15/P16 and P17/P18 respectively to obtain an upstream homologous arm fragment (772 bp) and a downstream homologous arm fragment (824 bp) from which TKT was knocked out. The amplified product was subjected to electrophoresis and purified by a column DNA gel recovery kit. The recovered DNA fragments were linked to a pK18mobsacB plasmid (Addgene) purified by Xbal I/BamH I enzyme digestion through an NEBuilder enzyme (NEB) at 50°C for 30 min. A monoclone which grew after transformation of the linking product was subjected to PCR identification by using primers M13F/M13R to obtain a positive knockout vector pK18-ΔTKT. This plasmid had kanamycin resistance as a screening marker. This plasmid was delivered for sequencing.

The correctly sequenced knockout plasmid pK18-ΔTKT was electrotransformed into *Corynebacterium glutamicum* YP097158 and wild type *Corynebacterium glutamicum* ATCC13032, and cultured in a medium for 30 h. Single colonies produced in culture were subjected to PCR identification by using primers P15/P18. The strains that amplified the stripes of 1596 bp and 3106 bp at the same time were both positive strains, and the strains that amplified a stripe of 3106 bp only were probiotics. The positive strains were screened on a 15% sucrose solid medium, and then cultured on a kanamycin-containing medium and a kanamycin-free medium for 30 h, respectively. The strains that grew on the kanamycin-free medium, but did not grow on the kanamycin-containing medium were selected, and further subjected to PCR identification by using primers P15/P18. The strains that amplified a stripe of 1596 bp were positive strains whose partial encoding region of the TKT gene was knocked out. The TKT fragments of the positive strains were subjected to PCR amplification again by using primers P15/P18 for sequencing. The correctly sequenced strains were named as YPL-tk -6 (the TKT gene on the genome of *Corynebacterium glutamicum* YP097158 was knocked out) and TKT-6 (the TKT gene on the genome of wild type *Corynebacterium glutamicum* ATCC13032 was knocked out).

### Example 7: L-lysine fermentation experiment

The strains constructed in Examples 2-6, the original strain YP097158 of *Corynebacterium glutamicum* and the wild type *Corynebacterium glutamicum* ATCC13032 were fermented in a fermentation tank of BLBIO-5GC-4-H model (Shanghai Bailun Biotechnology Co., Ltd.) using mediums shown in Table 5 and the control process shown in Table 6. After the fermentation, the L-lysine yield was detected by ninhydrin colorimetry. This process was repeated three times for each strain. The results were shown in Table 7.

**Table 5: Formulas of fermentation mediums**

| Components | Formula |
|---|---|
| Starch hydrolyzed sugar | 30 g/L |
| Ammonium sulfate | 12 g/L |
| Magnesium sulfate | 0.87 g/L |
| Molasses | 20 g/L |
| Acidified corn syrup | 3 mL/L |
| Phosphoric acid | 0.4 mL/L |
| Potassium chloride | 0.53 g/L |
| Defoamer (2% Paodi) | 4 mL/L |
| Ferrous sulfate | 120 mg/L |
| Manganese sulfate | 120 mg/L |
| Nicotinamide | 42 mg/L |
| Calcium pantothenate | 6.3 mg/L |
| Vitamin B1 | 6.3 mg/L |
| Copper and zinc salt solution | 0.6 g/L |
| Biotin | 0.88 mg/L |

**Table 6: Fermentation control process**

| Corrected to D0100% | Temperature of 37°C, air volume of 4 L/min, speed of 1000 rpm, tank pressure of 0 Mpa, and calibrated after 5 min | | |
|---|---|---|---|
| Inoculation amount | 10% | Culture temperature °C | 37°C |
| pH | pH6.9±0.05 | Dissolved oxygen DO | 10-30% |
| Initial conditions | | Temperature of 37°C, pH of 6.9, tank pressure of 0 Mpa, air volume of 3 L/min, and speed of 550 rpm | |
| Whole-process control | | whole-process control 1, the dissolved oxygen < 30%, and the speed was sequentially increased to 750 rpm→800 rpm→ air volume of 4 L/min→850 rpm→950 rpm; 2, the tank pressure was increased to 0.01 Mpa after 6 h of | |
| Residual sugar control | | 0.1-0.2% before F12h; residual sugar was controlled to 0.1-0.05% after F12h in combination with D0 requirements | |
| Ammonia-nitrogen control | | 0.1-0.15 before F12h; 0.15-0.25 for F12-F32h; 0.1-0.15 after F32h | |
| Material fed-batch: | | 25% of ammonia water, 70% of concentrated sugar, 50% of ammonium sulfate, and 10% of Paodi | |
| Fermentation cycle | | About 48 h | |

**Table 7: Yield and significance analysis of L-lysine in TKT engineered strains**

| Strains | L-lysine concentration (g/100mL) | | | Mean (g/L) | Significance analysis |
|---|---|---|---|---|---|
| | First fermentatio | Second fermentatio | Third fermentatio | | |
| ATCC13032 | 0.006 | 0.003 | 0.007 | 0.0053±0.0017 | |
| TKT-1 | 0.422 | 0.392 | 0.513 | 0.442±0.051 | P<0.01 |
| TKT-2 | 0.322 | 0.348 | 0.313 | 0.327±0.015 | P<0.01 |
| TKT-3 | 0.518 | 0.472 | 0.556 | 0.515±0.034 | P<0.01 |
| TKT-4 | 0.442 | 0.472 | 0.523 | 0.479±0.033 | P<0.01 |
| TKT-5 | 0.502 | 0.592 | 0.573 | 0.556±0.039 | P<0.01 |
| TKT-6 | 0.004 | 0.003 | 0.006 | 0.0043±0.0012 | Non-significant |
| YP097158 | 18.62 | 18.46 | 17.74 | 18.27±0.38 | |
| YPL-TKT-1 | 19.64 | 19.08 | 19.84 | 19.52±0.32 | P<0.01 |
| YPL-TKT-2 | 19.32 | 18.88 | 19.84 | 19.35±0.39 | P<0.05 |
| YPL-TKT-3 | 19.64 | 20.26 | 20.06 | 19.99±0.26 | P<0.01 |
| YPL-TKT-4 | 19.42 | 18.85 | 19.35 | 19.21±0.25 | P<0.05 |
| YPL-TKT-5 | 20.34 | 19.61 | 20.67 | 20.21±0.44 | P<0.01 |
| YPL-TKT-6 | 17.64 | 16.97 | 17.33 | 17.31±0.27 | P<0.01 |

The results were shown in Table 7. Point mutation (G979A) in the tkt gene encoding region and overexpression of the **TKT** or TKT^{A327T} gene in *Corynebacterium glutamicum* all contributed to an increase in L-lysine yield, while weakening or deletion of the TKT gene was not conducive to the accumulation of L-lysine.

The present invention is detailed above. For a person skilled in the art, the present invention may be implemented within a wide range under the same parameters, concentrations and conditions without departing from the purpose and scope of the present invention and without unnecessary experiments. Although special examples are given in the present invention, it should be understood that further improvements may be made to the present invention. In summary, according to the principles of the present invention, the present application is intended to include any change, use or improvement of the present invention, including changes that deviate from the scope disclosed in the present application and are made by conventional technologies known in the art. According to the scope of the claims attached below, some basic features can be applied.

### Industrial Applicability

The protein and the related biomaterial of the present invention can increase the yield of L-lysine and can be used for the production of L-lysine, and same have good application prospects.

The following sequences are involved in the present invention:
SEQ ID No. 1: wild type ORF(CDS) sequence (nucleotide sequence of 2103 bp) of TKT gene 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCGCTGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 2: TKT protein sequence (i.e., amino acid sequence 700aa encoded by SEQ ID No. 1) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERAAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 3: gene-mutant TKT^{A327T}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCACTGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 4: gene-mutant TKT^{A327T} protein sequence (i.e., amino acid sequence 700aa encoded by SEQ ID No. 3) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERTAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 5: gene-mutant TKT^{A327S}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCTCTGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 6: gene-mutant TKT^{A327S} protein sequence (i.e., amino acid sequence 700aa encoded by SEQ ID No. 5) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERSAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 7: gene-mutant TKT^{A327C}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCTGTGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 8: gene-mutant TKT^{A327C} protein sequence (i.e., amino acid sequence 700aa encoded by SEQ ID No. 7) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERCAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 9: gene-mutant TKT^{A327P}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCGCCCTGCACAGAAG
 
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 10: gene-mutant TKT^{A327P} protein sequence (i.e., amino acid sequence 700aa encoded by SEQ ID No. 9) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERPAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 11: gene-mutant TKT^{A327N}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCAATGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No. 12: gene-mutant TKT^{A327N} protein sequence (i.e., amino acid sequence 700aa encoded by SEQ ID No. 11) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERNAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No.13: gene-mutant TKT^{A327Q}ORF(CDS) sequence (nucleotide sequence of 2103 bp) 
TTGACCACCTTGACGCTGTCACCTGAACTTCAGGCGCTCACTGTACGCAATTACCCCTCTGATTGGTCCGATGTGGACACCAAGGCTGTA
GACACTGTTCGTGTCCTCGCTGCAGACGCTGTAGAAAACTGTGGCTCCGGCCACCCAGGCACCGCAATGAGCCTGGCTCCCCTTGCATAC
ACCTTGTACCAGCGGGTTATGAACGTAGATCCACAGGACACCAACTGGGCAGGCCGTGACCGCTTCGTTCTTTCTTGTGGCCACTCCTCT
TTGACCCAGTACATCCAGCTTTACTTGGGTGGATTCGGCCTTGAGATGGATGACCTGAAGGCTCTGCGCACCTGGGATTCCTTGACCCCA
GGACACCCTGAGTACCGCCACACCAAGGGCGTTGAGATCACCACTGGCCCTCTTGGCCAGGGTCTTGCATCTGCAGTTGGTATGGCCATG
GCTGCTCGTCGTGAGCGTGGCCTATTCGACCCAACCGCTGCTGAGGGCGAATCCCCATTCGACCACCACATCTACGTCATTGCTTCTGAT
GGTGACCTGCAGGAAGGTGTCACCTCTGAGGCATCCTCCATCGCTGGCACCCAGCAGCTGGGCAACCTCATCGTGTTCTGGGATGACAAC
CGCATCTCCATCGAAGACAACACTGAGATCGCTTTCAACGAGGACGTTGTTGCTCGTTACAAGGCTTACGGCTGGCAGACCATTGAGGTT
GAGGCTGGCGAGGACGTTGCAGCAATCGAAGCTGCAGTGGCTGAGGCTAAGAAGGACACCAAGCGACCTACCTTCATCCGCGTTCGCACC
ATCATCGGCTTCCCAGCTCCAACTATGATGAACACCGGTGCTGTGCACGGTGCTGCTCTTGGCGCAGCTGAGGTTGCAGCAACCAAGACT
GAGCTTGGATTCGATCCTGAGGCTCACTTCGCGATCGACGATGAGGTTATCGCTCACACCCGCTCCCTCGCAGAGCGCCAGGCACAGAAG
AAGGCTGCATGGCAGGTCAAGTTCGATGAGTGGGCAGCTGCCAACCCTGAGAACAAGGCTCTGTTCGATCGCCTGAACTCCCGTGAGCTT
CCAGCGGGCTACGCTGACGAGCTCCCAACATGGGATGCAGATGAGAAGGGCGTCGCAACTCGTAAGGCTTCCGAGGCTGCACTTCAGGCA
CTGGGCAAGACCCTTCCTGAGCTGTGGGGCGGTTCCGCTGACCTCGCAGGTTCCAACAACACCGTGATCAAGGGCTCCCCTTCCTTCGGC
CCTGAGTCCATCTCCACCGAGACCTGGTCTGCTGAGCCTTACGGCCGTAACCTGCACTTCGGTATCCGTGAGCACGCTATGGGATCCATC
CTCAACGGCATTTCCCTCCACGGTGGCACCCGCCCATACGGCGGAACCTTCCTCATCTTCTCCGACTACATGCGTCCTGCAGTTCGTCTT
GCAGCTCTCATGGAGACCGACGCTTACTACGTCTGGACCCACGACTCCATCGGTCTGGGCGAAGATGGCCCAACCCACCAGCCTGTTGAA
ACCTTGGCTGCACTGCGCGCCATCCCAGGTCTGTCCGTCCTGCGTCCTGCAGATGCGAACGAGACCGCCCAGGCTTGGGCTGCAGCACTT
GAGTACAAGGAAGGCCCTAAGGGTCTTGCACTGACCCGCCAGAACGTTCCTGTTCTGGAAGGCACCAAGGAGAAGGCTGCTGAAGGCGTT
CGCCGCGGTGGCTACGTCCTGGTTGAGGGTTCCAAGGAAACCCCAGATGTGATCCTCATGGGCTCCGGCTCCGAGGTTCAGCTTGCAGTT
AACGCTGCGAAGGCTCTGGAAGCTGAGGGCGTTGCAGCTCGCGTTGTTTCCGTTCCTTGCATGGATTGGTTCCAGGAGCAGGACGCAGAG
TACATCGAGTCCGTTCTGCCTGCAGCTGTGACCGCTCGTGTGTCTGTTGAAGCTGGCATCGCAATGCCTTGGTACCGCTTCTTGGGCACC
CAGGGCCGTGCTGTCTCCCTTGAGCACTTCGGTGCTTCTGCGGATTACCAGACCCTGTTTGAGAAGTTCGGCATCACCACCGATGCAGTC
GTGGCAGCGGCCAAGGACTCCATTAACGGTTAA
SEQ ID No.14: gene-mutant TKT^{A327Q} protein sequence (i.e., amino acid sequence 700aa encoded by SEQ ID No. 13) 
MTTLTLSPELQALTVRNYPSDWSDVDTKAVDTVRVLAADAVENCGSGHPGTAMSLAPLAYTLYQRVMNVDPQDTNWAGRDRFVLSCGHSS
LTQYIQLYLGGFGLEMDDLKALRTWDSLTPGHPEYRHTKGVEITTGPLGQGLASAVGMAMAARRERGLFDPTAAEGESPFDHHIYVIASD
GDLQEGVTSEASSIAGTQQLGNLIVFWDDNRISIEDNTEIAFNEDVVARYKAYGWQTIEVEAGEDVAAIEAAVAEAKKDTKRPTFIRVRT
IIGFPAPTMMNTGAVHGAALGAAEVAATKTELGFDPEAHFAIDDEVIAHTRSLAERQAQKKAAWQVKFDEWAAANPENKALFDRLNSREL
PAGYADELPTWDADEKGVATRKASEAALQALGKTLPELWGGSADLAGSNNTVIKGSPSFGPESISTETWSAEPYGRNLHFGIREHAMGSI
LNGISLHGGTRPYGGTFLIFSDYMRPAVRLAALMETDAYYVWTHDSIGLGEDGPTHQPVETLAALRAIPGLSVLRPADANETAQAWAAAL
EYKEGPKGLALTRQNVPVLEGTKEKAAEGVRRGGYVLVEGSKETPDVILMGSGSEVQLAVNAAKALEAEGVAARVVSVPCMDWFQEQDAE
YIESVLPAAVTARVSVEAGIAMPWYRFLGTQGRAVSLEHFGASADYQTLFEKFGITTDAVVAAAKDSING
SEQ ID No. 15: primer TKT-F/TKT-R amplification sequence (a size of 2689 bp) 
CAGAATAATTAAGCTTGCATGCCTGCAGGTCGACTTCACAGCGGACGATTTCAGGCCCTCGTAGCTCGAGAGTTTGAAGGGGTCCGATTC
GTTCCGTTCGTGACGCTTTGTGAGGTTTTTTGACGTTGCACCGTATTGCTTGCCGAACATTTTTCTTTTCCTTTCGGTTTTTCGAGAATT
TTCACCTACAAAAGCCCACGTCACAGCTCCCAGACTTAAGATTGATCACACCTTTGACACATTTGAACCACAGTTGGTTATAAAATGGGT
TCAACATCACTATGGTTAGAGGTGTTGACGGGTCAGATTAAGCAAAGACTACTTTCGGGGTAGATCACCTTTGCCAAATTTGAACCAATT
AACCTAAGTCGTAGATCTGATCATCGGATCTAACGAAAACGAACCAAAACTTTGGTCCCGGTTTAACCCAGGAAGGATTGACCACCTTGA

CGCTGTCACCTGAACTTCAGGCGCTCACTGTACGCAATTACCCCTCTGATTGGTCCGATGTGGACACCAAGGCTGTAGACACTGTTCGTG
TCCTCGCTGCAGACGCTGTAGAAAACTGTGGCTCCGGCCACCCAGGCACCGCAATGAGCCTGGCTCCCCTTGCATACACCTTGTACCAGC
GGGTTATGAACGTAGATCCACAGGACACCAACTGGGCAGGCCGTGACCGCTTCGTTCTTTCTTGTGGCCACTCCTCTTTGACCCAGTACA
TCCAGCTTTACTTGGGTGGATTCGGCCTTGAGATGGATGACCTGAAGGCTCTGCGCACCTGGGATTCCTTGACCCCAGGACACCCTGAGT
ACCGCCACACCAAGGGCGTTGAGATCACCACTGGCCCTCTTGGCCAGGGTCTTGCATCTGCAGTTGGTATGGCCATGGCTGCTCGTCGTG
AGCGTGGCCTATTCGACCCAACCGCTGCTGAGGGCGAATCCCCATTCGACCACCACATCTACGTCATTGCTTCTGATGGTGACCTGCAGG
AAGGTGTCACCTCTGAGGCATCCTCCATCGCTGGCACCCAGCAGCTGGGCAACCTCATCGTGTTCTGGGATGACAACCGCATCTCCATCG
AAGACAACACTGAGATCGCTTTCAACGAGGACGTTGTTGCTCGTTACAAGGCTTACGGCTGGCAGACCATTGAGGTTGAGGCTGGCGAGG
ACGTTGCAGCAATCGAAGCTGCAGTGGCTGAGGCTAAGAAGGACACCAAGCGACCTACCTTCATCCGCGTTCGCACCATCATCGGCTTCC
CAGCTCCAACTATGATGAACACCGGTGCTGTGCACGGTGCTGCTCTTGGCGCAGCTGAGGTTGCAGCAACCAAGACTGAGCTTGGATTCG
ATCCTGAGGCTCACTTCGCGATCGACGATGAGGTTATCGCTCACACCCGCTCCCTCGCAGAGCGCGCTGCACAGAAGAAGGCTGCATGGC
AGGTCAAGTTCGATGAGTGGGCAGCTGCCAACCCTGAGAACAAGGCTCTGTTCGATCGCCTGAACTCCCGTGAGCTTCCAGCGGGCTACG
CTGACGAGCTCCCAACATGGGATGCAGATGAGAAGGGCGTCGCAACTCGTAAGGCTTCCGAGGCTGCACTTCAGGCACTGGGCAAGACCC
TTCCTGAGCTGTGGGGCGGTTCCGCTGACCTCGCAGGTTCCAACAACACCGTGATCAAGGGCTCCCCTTCCTTCGGCCCTGAGTCCATCT
CCACCGAGACCTGGTCTGCTGAGCCTTACGGCCGTAACCTGCACTTCGGTATCCGTGAGCACGCTATGGGATCCATCCTCAACGGCATTT
CCCTCCACGGTGGCACCCGCCCATACGGCGGAACCTTCCTCATCTTCTCCGACTACATGCGTCCTGCAGTTCGTCTTGCAGCTCTCATGG
AGACCGACGCTTACTACGTCTGGACCCACGACTCCATCGGTCTGGGCGAAGATGGCCCAACCCACCAGCCTGTTGAAACCTTGGCTGCAC
TGCGCGCCATCCCAGGTCTGTCCGTCCTGCGTCCTGCAGATGCGAACGAGACCGCCCAGGCTTGGGCTGCAGCACTTGAGTACAAGGAAG
GCCCTAAGGGTCTTGCACTGACCCGCCAGAACGTTCCTGTTCTGGAAGGCACCAAGGAGAAGGCTGCTGAAGGCGTTCGCCGCGGTGGCT
ACGTCCTGGTTGAGGGTTCCAAGGAAACCCCAGATGTGATCCTCATGGGCTCCGGCTCCGAGGTTCAGCTTGCAGTTAACGCTGCGAAGG
CTCTGGAAGCTGAGGGCGTTGCAGCTCGCGTTGTTTCCGTTCCTTGCATGGATTGGTTCCAGGAGCAGGACGCAGAGTACATCGAGTCCG
TTCTGCCTGCAGCTGTGACCGCTCGTGTGTCTGTTGAAGCTGGCATCGCAATGCCTTGGTACCGCTTCTTGGGCACCCAGGGCCGTGCTG
TCTCCCTTGAGCACTTCGGTGCTTCTGCGGATTACCAGACCCTGTTTGAGAAGTTCGGCATCACCACCGATGCAGTCGTGGCAGCGGCCA
AGGACTCCATTAACGGTTAATTGCCCTGCTGTTTTTAGCTTCAACCCGGGGCAATATGATTCTCCGGAATTTTATTGCCCCGGGTTGTTG
TTGTTAATCGGTACAAAGGGTCTTAAGCACATCCCTTACTTGCCTGGTACCGAGCTCGAATTCAGCTTGGCTGTTTTGG
SEQ ID No. 16: genome-integrated P7/P8TKT and its promoter sequence (2621 bp) 
TTCACAGCGGACGATTTCAGGCCCTCGTAGCTCGAGAGTTTGAAGGGGTCCGATTCGTTCCGTTCGTGACGCTTTGTGAGGTTTTTTGAC
GTTGCACCGTATTGCTTGCCGAACATTTTTCTTTTCCTTTCGGTTTTTCGAGAATTTTCACCTACAAAAGCCCACGTCACAGCTCCCAGA
CTTAAGATTGATCACACCTTTGACACATTTGAACCACAGTTGGTTATAAAATGGGTTCAACATCACTATGGTTAGAGGTGTTGACGGGTC
AGATTAAGCAAAGACTACTTTCGGGGTAGATCACCTTTGCCAAATTTGAACCAATTAACCTAAGTCGTAGATCTGATCATCGGATCTAAC
GAAAACGAACCAAAACTTTGGTCCCGGTTTAACCCAGGAAGGATTGACCACCTTGACGCTGTCACCTGAACTTCAGGCGCTCACTGTACG
CAATTACCCCTCTGATTGGTCCGATGTGGACACCAAGGCTGTAGACACTGTTCGTGTCCTCGCTGCAGACGCTGTAGAAAACTGTGGCTC
CGGCCACCCAGGCACCGCAATGAGCCTGGCTCCCCTTGCATACACCTTGTACCAGCGGGTTATGAACGTAGATCCACAGGACACCAACTG
GGCAGGCCGTGACCGCTTCGTTCTTTCTTGTGGCCACTCCTCTTTGACCCAGTACATCCAGCTTTACTTGGGTGGATTCGGCCTTGAGAT
GGATGACCTGAAGGCTCTGCGCACCTGGGATTCCTTGACCCCAGGACACCCTGAGTACCGCCACACCAAGGGCGTTGAGATCACCACTGG
CCCTCTTGGCCAGGGTCTTGCATCTGCAGTTGGTATGGCCATGGCTGCTCGTCGTGAGCGTGGCCTATTCGACCCAACCGCTGCTGAGGG
CGAATCCCCATTCGACCACCACATCTACGTCATTGCTTCTGATGGTGACCTGCAGGAAGGTGTCACCTCTGAGGCATCCTCCATCGCTGG
CACCCAGCAGCTGGGCAACCTCATCGTGTTCTGGGATGACAACCGCATCTCCATCGAAGACAACACTGAGATCGCTTTCAACGAGGACGT
TGTTGCTCGTTACAAGGCTTACGGCTGGCAGACCATTGAGGTTGAGGCTGGCGAGGACGTTGCAGCAATCGAAGCTGCAGTGGCTGAGGC
TAAGAAGGACACCAAGCGACCTACCTTCATCCGCGTTCGCACCATCATCGGCTTCCCAGCTCCAACTATGATGAACACCGGTGCTGTGCA
CGGTGCTGCTCTTGGCGCAGCTGAGGTTGCAGCAACCAAGACTGAGCTTGGATTCGATCCTGAGGCTCACTTCGCGATCGACGATGAGGT
TATCGCTCACACCCGCTCCCTCGCAGAGCGCGCTGCACAGAAGAAGGCTGCATGGCAGGTCAAGTTCGATGAGTGGGCAGCTGCCAACCC
TGAGAACAAGGCTCTGTTCGATCGCCTGAACTCCCGTGAGCTTCCAGCGGGCTACGCTGACGAGCTCCCAACATGGGATGCAGATGAGAA
GGGCGTCGCAACTCGTAAGGCTTCCGAGGCTGCACTTCAGGCACTGGGCAAGACCCTTCCTGAGCTGTGGGGCGGTTCCGCTGACCTCGC
AGGTTCCAACAACACCGTGATCAAGGGCTCCCCTTCCTTCGGCCCTGAGTCCATCTCCACCGAGACCTGGTCTGCTGAGCCTTACGGCCG
TAACCTGCACTTCGGTATCCGTGAGCACGCTATGGGATCCATCCTCAACGGCATTTCCCTCCACGGTGGCACCCGCCCATACGGCGGAAC
CTTCCTCATCTTCTCCGACTACATGCGTCCTGCAGTTCGTCTTGCAGCTCTCATGGAGACCGACGCTTACTACGTCTGGACCCACGACTC
CATCGGTCTGGGCGAAGATGGCCCAACCCACCAGCCTGTTGAAACCTTGGCTGCACTGCGCGCCATCCCAGGTCTGTCCGTCCTGCGTCC
TGCAGATGCGAACGAGACCGCCCAGGCTTGGGCTGCAGCACTTGAGTACAAGGAAGGCCCTAAGGGTCTTGCACTGACCCGCCAGAACGT
TCCTGTTCTGGAAGGCACCAAGGAGAAGGCTGCTGAAGGCGTTCGCCGCGGTGGCTACGTCCTGGTTGAGGGTTCCAAGGAAACCCCAGA
TGTGATCCTCATGGGCTCCGGCTCCGAGGTTCAGCTTGCAGTTAACGCTGCGAAGGCTCTGGAAGCTGAGGGCGTTGCAGCTCGCGTTGT
TTCCGTTCCTTGCATGGATTGGTTCCAGGAGCAGGACGCAGAGTACATCGAGTCCGTTCTGCCTGCAGCTGTGACCGCTCGTGTGTCTGT
TGAAGCTGGCATCGCAATGCCTTGGTACCGCTTCTTGGGCACCCAGGGCCGTGCTGTCTCCCTTGAGCACTTCGGTGCTTCTGCGGATTA
CCAGACCCTGTTTGAGAAGTTCGGCATCACCACCGATGCAGTCGTGGCAGCGGCCAAGGACTCCATTAACGGTTAATTGCCCTGCTGTTT
TTAGCTTCAACCCGGGGCAATATGATTCTCCGGAATTTTATTGCCCCGGGTTGTTGTTGTTAATCGGTACAAAGGGTCTTAAGCACATCC
CTTACTTGCCT
SEQ ID No. 17: genome-integrated P7/P8 TKT^{A327T} and its promoter sequence (2621 bp)

TTCACAGCGGACGATTTCAGGCCCTCGTAGCTCGAGAGTTTGAAGGGGTCCGATTCGTTCCGTTCGTGACGCTTTGTGAGGTTTTTTGAC
GTTGCACCGTATTGCTTGCCGAACATTTTTCTTTTCCTTTCGGTTTTTCGAGAATTTTCACCTACAAAAGCCCACGTCACAGCTCCCAGA
CTTAAGATTGATCACACCTTTGACACATTTGAACCACAGTTGGTTATAAAATGGGTTCAACATCACTATGGTTAGAGGTGTTGACGGGTC
AGATTAAGCAAAGACTACTTTCGGGGTAGATCACCTTTGCCAAATTTGAACCAATTAACCTAAGTCGTAGATCTGATCATCGGATCTAAC
GAAAACGAACCAAAACTTTGGTCCCGGTTTAACCCAGGAAGGATTGACCACCTTGACGCTGTCACCTGAACTTCAGGCGCTCACTGTACG
CAATTACCCCTCTGATTGGTCCGATGTGGACACCAAGGCTGTAGACACTGTTCGTGTCCTCGCTGCAGACGCTGTAGAAAACTGTGGCTC
CGGCCACCCAGGCACCGCAATGAGCCTGGCTCCCCTTGCATACACCTTGTACCAGCGGGTTATGAACGTAGATCCACAGGACACCAACTG
GGCAGGCCGTGACCGCTTCGTTCTTTCTTGTGGCCACTCCTCTTTGACCCAGTACATCCAGCTTTACTTGGGTGGATTCGGCCTTGAGAT
GGATGACCTGAAGGCTCTGCGCACCTGGGATTCCTTGACCCCAGGACACCCTGAGTACCGCCACACCAAGGGCGTTGAGATCACCACTGG
CCCTCTTGGCCAGGGTCTTGCATCTGCAGTTGGTATGGCCATGGCTGCTCGTCGTGAGCGTGGCCTATTCGACCCAACCGCTGCTGAGGG
CGAATCCCCATTCGACCACCACATCTACGTCATTGCTTCTGATGGTGACCTGCAGGAAGGTGTCACCTCTGAGGCATCCTCCATCGCTGG
CACCCAGCAGCTGGGCAACCTCATCGTGTTCTGGGATGACAACCGCATCTCCATCGAAGACAACACTGAGATCGCTTTCAACGAGGACGT
TGTTGCTCGTTACAAGGCTTACGGCTGGCAGACCATTGAGGTTGAGGCTGGCGAGGACGTTGCAGCAATCGAAGCTGCAGTGGCTGAGGC
TAAGAAGGACACCAAGCGACCTACCTTCATCCGCGTTCGCACCATCATCGGCTTCCCAGCTCCAACTATGATGAACACCGGTGCTGTGCA
CGGTGCTGCTCTTGGCGCAGCTGAGGTTGCAGCAACCAAGACTGAGCTTGGATTCGATCCTGAGGCTCACTTCGCGATCGACGATGAGGT
TATCGCTCACACCCGCTCCCTCGCAGAGCGCACTGCACAGAAGAAGGCTGCATGGCAGGTCAAGTTCGATGAGTGGGCAGCTGCCAACCC
TGAGAACAAGGCTCTGTTCGATCGCCTGAACTCCCGTGAGCTTCCAGCGGGCTACGCTGACGAGCTCCCAACATGGGATGCAGATGAGAA
GGGCGTCGCAACTCGTAAGGCTTCCGAGGCTGCACTTCAGGCACTGGGCAAGACCCTTCCTGAGCTGTGGGGCGGTTCCGCTGACCTCGC
AGGTTCCAACAACACCGTGATCAAGGGCTCCCCTTCCTTCGGCCCTGAGTCCATCTCCACCGAGACCTGGTCTGCTGAGCCTTACGGCCG
TAACCTGCACTTCGGTATCCGTGAGCACGCTATGGGATCCATCCTCAACGGCATTTCCCTCCACGGTGGCACCCGCCCATACGGCGGAAC
CTTCCTCATCTTCTCCGACTACATGCGTCCTGCAGTTCGTCTTGCAGCTCTCATGGAGACCGACGCTTACTACGTCTGGACCCACGACTC
CATCGGTCTGGGCGAAGATGGCCCAACCCACCAGCCTGTTGAAACCTTGGCTGCACTGCGCGCCATCCCAGGTCTGTCCGTCCTGCGTCC
TGCAGATGCGAACGAGACCGCCCAGGCTTGGGCTGCAGCACTTGAGTACAAGGAAGGCCCTAAGGGTCTTGCACTGACCCGCCAGAACGT
TCCTGTTCTGGAAGGCACCAAGGAGAAGGCTGCTGAAGGCGTTCGCCGCGGTGGCTACGTCCTGGTTGAGGGTTCCAAGGAAACCCCAGA
TGTGATCCTCATGGGCTCCGGCTCCGAGGTTCAGCTTGCAGTTAACGCTGCGAAGGCTCTGGAAGCTGAGGGCGTTGCAGCTCGCGTTGT
TTCCGTTCCTTGCATGGATTGGTTCCAGGAGCAGGACGCAGAGTACATCGAGTCCGTTCTGCCTGCAGCTGTGACCGCTCGTGTGTCTGT
TGAAGCTGGCATCGCAATGCCTTGGTACCGCTTCTTGGGCACCCAGGGCCGTGCTGTCTCCCTTGAGCACTTCGGTGCTTCTGCGGATTA
CCAGACCCTGTTTGAGAAGTTCGGCATCACCACCGATGCAGTCGTGGCAGCGGCCAAGGACTCCATTAACGGTTAATTGCCCTGCTGTTT
TTAGCTTCAACCCGGGGCAATATGATTCTCCGGAATTTTATTGCCCCGGGTTGTTGTTGTTAATCGGTACAAAGGGTCTTAAGCACATCC
CTTACTTGCCT

## Claims

1. A protein, which is A1) or A2) or A3) as follows:
A1) a protein comprising SEQ ID No. 2, or a mutant protein which is obtained by mutating an alanine residue at position 327 in SEQ ID No. 2 into a threonine residue, a serine residue, a cysteine residue, a proline residue, an asparagine residue, a glutamine residue, a phenylalanine residue, a leucine residue, a valine residue, an isoleucine residue, an aspartic acid residue, a methionine residue, an arginine residue, a glutamic acid residue, a glycine residue, a histidine residue, a lysine residue, a tryptophan residue, or a tyrosine residue;
A2) a protein which is obtained by substitution and/or deletion and/or addition of one or more amino acid residues in an amino acid sequence of the protein of A1) except for position 327 and has the same function as A1); and
A3) a fusion protein obtained by linking a tag to an N-terminal or/and a C-terminal of A1) or A2).

2. A biomaterial associated with the protein according to claim 1, which is any of B1) to B4) as follows:
B1) a nucleic acid molecule that encodes the protein according to claim 1;
B2) an expression cassette containing the nucleic acid molecule of B1);
B3) a recombinant vector containing the nucleic acid molecule of B1), or a recombinant vector containing the expression cassette of B2); and
B4) a recombinant microorganism containing the nucleic acid molecule of B1), or a recombinant microorganism containing the expression cassette of B2, or a recombinant microorganism containing the recombinant vector of B3).

3. The biomaterial according to claim 2, wherein the nucleic acid molecule of B1) is any of b11)-b19) as follows:
b11) a DNA molecule as represented by SEQ ID No. 3 in a sequence listing;
b12) a DNA molecule as represented by SEQ ID No. 5 in the sequence listing;
b13) a DNA molecule as represented by SEQ ID No. 7 in the sequence listing;
b14) a DNA molecule as represented by SEQ ID No. 9 in the sequence listing;
b15) a DNA molecule as represented by SEQ ID No. 11 in the sequence listing;
b16) a DNA molecule as represented by SEQ ID No. 13 in the sequence listing;
b17) a DNA molecule as represented by SEQ ID No. 1 in the sequence listing;
b18) a DNA molecule that has the identity of 75% or more with a nucleotide sequence defined by any of b11)- b17) and encodes the protein according to claim 1; and
b19) a genomic DNA molecule that hybridizes with the nucleotide sequence defined by any of b11)- b18) under stringent conditions and encodes the protein according to claim 1.

4. The biomaterial according to claim 2 or 3, wherein a promoter in the expression cassette of B2) is a DNA molecule shown by positions 35-437 in SEQ ID No. 15; and
the recombinant microorganism of B4) is a recombinant microorganism which is obtained by replacing a TKT gene in the microorganism containing the tkt gene as represented by SEQ ID No. 1 with that as represented by SEQ ID No. 3, SEQ ID No. 5, SEQ ID No. 7, SEQ ID No. 9, SEQ ID No. 11 or SEQ ID No. 13, or a recombinant microorganism which is obtained by introducing the nucleic acid molecule of B1) into the microorganism and expressing the nucleic acid molecule of B1).

5. A method for preparing L-lysine, comprising: expressing the protein according to claim 1 in a receptor biological cell, or increasing the content or activity of the protein according to claim 1 in the receptor biological cell, or increasing the content or activity of the protein as represented by SEQ ID No. 4 or SEQ ID No. 6 or SEQ ID No. 8 or SEQ ID No. 10 or SEQ ID No. 12 or SEQ ID No. 14 or SEQ ID No. 2 in the receptor biological cell to obtain a recombinant biological cell; and culturing the recombinant biological cell to obtain the L-lysine.

6. The method according to claim 5, wherein the biological cell is yeast, bacteria, algae, fungus, a plant cell or an animal cell that is able to synthesize the L-lysine.

7. The method according to claim 6, wherein the bacteria are *Corynebacterium glutamicum.*

8. A product for preparing L-lysine, which contains the protein according to claim 1 or the biomaterial according to any of claims 2 to 4.

9. An application of the protein according to claim 1 in the production of L-lysine, or in the preparation of a product for producing L-lysine.

10. An application of the biomaterial according to any of claims 2 to 4 in the production of L-lysine, or in the preparation of a product for producing L-lysine.

11. An application of the protein according to claim 1 in the preparation of food, feed or medicine containing L-lysine.

12. An application of the biomaterial according to any of claims 2 to 4 in the preparation of food, feed or medicine containing L-lysine.

13. An application of the method according to any of claims 5 to 7 in the preparation of food, feed or medicine containing L-lysine.

14. An application of the product according to claim 8 in the preparation of food, feed or medicine containing L-lysine.
